# EUROPEAN PATENT SPECIFICATION

(11) **EP 4 618 841 B1**
(45) Date of publication and mention of the grant of the patent: **05.08.2026**
(21) Application number: 23804698.1
(22) Date of filing: 13.11.2023
(51) Int. Cl.: A61B 5/1486

(54) **MEDICAL DEVICE FOR DETECTING AT LEAST ONE ANALYTE IN A BODY FLUID**
MEDIZINISCHE VORRICHTUNG ZUM NACHWEIS VON MINDESTENS EINEM ANALYTEN IN EINER KÖRPERFLÜSSIGKEIT
DISPOSITIF MÉDICAL POUR DÉTECTER AU MOINS UN ANALYTE DANS UN FLUIDE CORPOREL

(30) Priority: 14.11.2022 EP 22207361
(43) Date of publication of application: 24.09.2025
(73) Proprietor: F. Hoffmann-La Roche AG, 4070 Basel (CH); Roche Diabetes Care GmbH, 68305 Mannheim (DE)
(72) Inventor: BOOTZ, Felix, 68305 Mannheim (DE)
(74) Representative: Pfaffmann, Lucas
(86) International application number: PCT/EP2023/081553
(87) International publication number: WO 2024/104935

(56) References cited:
- WO-A1-2009/139522
- US-A1- 2021 307 657

## Description

The present invention relates to a medical device for detecting at least one analyte in a body fluid, comprising a base body that is configured for reversibly attaching the medical device to a patient's body; an insertable analyte sensor comprising an insertable portion that is configured for being at least partially inserted into body tissue of a user; and an electronics unit that is operably connected to the insertable analyte sensor, wherein the insertable analyte sensor comprises a first electrode and a second electrode, both, forming part of the insertable portion, wherein the first electrode comprises a first free end and a second end, wherein the second end of the first electrode is electrically connected to a first electric conductor electrically connecting the first electrode of the insertable analyte sensor to the electronics unit, wherein the second electrode comprises a first free end and a second end, wherein the second end of the second electrode is electrically connected to a second electric conductor electrically connecting the second electrode of the insertable analyte sensor to the electronics unit, and wherein the first electrode extending from the second end towards its first free end and the second electrode extending from the second end towards its first free end are configured to be, during use of the medical device, at least partially in electric contact with the body tissue of a user.

A hitherto known medical device for detecting at least one analyte in a body fluid is, during use, removably attached to a user's body, wherein the insertable analyte sensor is inserted into the body tissue of the user. Accordingly, the first electrode and the second electrode are in direct contact with the body tissue, and electrically connected to the electronics unit. Thus, failure of the electronics unit, such a short circuit, may result in a high electric current flowing into the user's body via the first electrode and the second electrode. Therefore, in order increase the user's safety known medical devices comprises redundant electronic components such that a failure of an electronic component does not result in a high electric current flowing into the user's body, but is compensated by the respective redundant component. However, the number of extra, redundant electric components increase the production costs and results in a larger size of the medical device.

US 2021/0307657 A1 refers to pre-connected analyte sensors. A pre-connected analyte sensor includes a sensor carrier attached to an analyte sensor. The sensor carrier includes a substrate configured to mechanical coupling of the sensor to testing, calibration, or wearable equipment. The sensor carrier also includes conductive contacts for electrically coupling sensor electrodes to the testing, calibration, or wearable equipment.

WO 2009/139522 A1 refers to an apparatus and method for measuring blood-glucose using an electrophoresis phenomenon. The non-invasive blood-glucose measuring apparatus using an electrophoresis phenomenon includes: two different extraction electrodes; an extraction alternating unit applying constant current to one of the two extraction electrodes; and a microprocessor generating a control signal for selecting one of extraction electrodes.

Accordingly, it is an object of the present invention to provide a medical device for detecting at least one analyte in a body fluid which avoid at least one disadvantage of hitherto known medical devices. In particular, it is an object of the present invention to provide a medical device for detecting at least one analyte in a body fluid mitigating a risk of a high current flowing into the body tissue of a user while allowing for lower production costs.

At least one of the above objects is solved by the provision of the subject matter disclosed in the claims and throughout the specification.

According to a first aspect, a medical device for detecting at least one analyte in a body fluid, is provided, the medical device comprising a base body that is configured for reversibly attaching the medical device to a patient's body; an insertable analyte sensor comprising an insertable portion that is configured for being at least partially inserted into body tissue of a user; and an electronics unit that is operably connected to the insertable analyte sensor, wherein the insertable analyte sensor comprises a first electrode and a second electrode, both, forming part of the insertable portion, wherein the first electrode comprises a first free end and a second end, wherein the second end of the first electrode is electrically connected to a first electric conductor electrically connecting the first electrode of the insertable analyte sensor to the electronics unit, wherein the second electrode comprises a first free end and a second end, wherein the second end of the second electrode is electrically connected to a second electric conductor electrically connecting the second electrode of the insertable analyte sensor to the electronics unit, and wherein the first electrode extending from the second end towards its first free end and the second electrode extending from the second end towards its first free end are configured to be, during use of the medical device, at least partially in electric contact with the body tissue of a user, wherein the electrical resistance of the first electric conductor and the electrical resistance of the second electric conductor is higher than the electrical resistance of the first electrode and the electrical resistance of the second electrode.

The medical device for detecting at least one analyte in a body fluid can be removably attached to a user's body. For instance, the base body can be adhered to a user's skin. For example, the base body can comprise an adhesive layer or can comprise a patch comprising an adhesive that is configured for removably adhering the medical device to a user's body.

When being attached to a user's body, the insertable portion of the insertable analyte sensor is inserted into the body tissue of the user, wherein the first electrode and the second electrode are in electric contact with the body tissue and in electric contact with the electronics unit via the first conductor and the second conductor, respectively. Presence of an analyte in the body tissue will affect an electric current flowing between the first electrode and the second electrode. The change in electric current can be evaluated by the electronics unit so to allow a detection of the respective analyte. By way of example, only, the electronics unit can be a printed circuit board.

The analyte can be one or more analytes, preferably metabolites such as glucose, lactate, triglycerides, cholesterol or analytes in bodily fluids, such as for example blood or interstitial fluid or other bodily fluids. For instance, the insertable analyte sensor can be an electrochemical sensor that is configured for detecting at least one analyte, wherein the first electrode is configured as a working electrode and is used as transducer, and the second electrode is configured as a counter electrode.

The medical device can comprise a power supply that is configured for providing electric power to the electronics unit. The power supply can be a battery or a rechargeable battery that is configured for providing electric power to the electronics unit. By way of example, only, the battery can be configured to provide an electric power of 3.4 V.

In order to mitigate a risk of an uncontrolled electric current flowing into the user's body in case of a failure of the electronics unit or a component thereof, the electrical resistance of the first conductor and the electrical resistance of the second conductor is higher than the electrical resistance of the first electrode and the electrical resistance of the second electrode. During use, an electric current is flowing between the first electrode and the second electrode, the first electric conductor and the second electric conductor together with the first electrode and the second electrode form a potentiometer. The higher electrical resistance of the first conductor and the second conductor limits the electric current flowing through the first conductor and the second conductor, so that the electric current that is flowing through the first electrode and the second electrode is limited, too. Consequently, there is no need for redundant electric components that would limit the electric current flowing through the first electrode and the second electrode in case of a failure of the electronics unit or parts thereof. Without the need of redundant electric components the overall number of electronic components of the electronics unit can be reduced, such that the medical device can be of smaller size and with lower production costs.

According to an embodiment, the sum of the electrical resistance of first electric conductor and the electrical resistance of the second electric conductor is higher than the sum of the electrical resistance of the first electrode and the electrical resistance of the second electrode. For instance, the sum of the electrical resistance of the first electrode and the electrical resistance of the second electrode can be 9 kOhm, while the sum of the electrical resistance of the first electrical conductor and the electrical resistance of the second electrical conductor can be 90 kOhm.

The electrical resistance of the first electrode and the electrical resistance of the second electrode can be equally high, and/or that the electrical resistance of the first electric conductor and the electrical resistance of the second electric conductor can be equally high. For the ease of its production, and for obtaining a symmetric potentiometer, it is beneficial that the electrical resistance of the first electrode is equal to the electrical resistance of the second electrode. Likewise, the electrical resistance of the first conductor is equal to the electrical resistance of the second conductor. In the meaning of the present invention, equal means identical measurement values that, however, may deviate by a common measuring inaccuracy and production inaccuracy.

In an embodiment, the electrical resistance of the first electric conductor and the electrical resistance of the second electric conductor is at least 2-times, at least 4-times, at least 5-times or at least 10-times higher than the electrical resistance of the first electrode and the electrical resistance of the second electrode. For instance, the electrical resistance of the first electrode and electrical resistance of the second electrode each can be 9 kOhm, while the electrical resistance of the first electric conductor and the electrical resistance of the second electric conductor each can be 90 kOhm.

In case of a failure of the electronics unit or parts thereof, an electric current that is uncontrolled flowing via the first electrode and the second electrode into body tissue, may harm the body tissue if the current exceeds 50 µA. Therefore, the electrical resistance of the first electric conductor and the electrical resistance of the second electric conductor, that during use limit a maximum electric current flowing through the first electrode and the second electrode, are selected so that the voltage of the power supply divided by the sum of the electrical resistance of the first electrical conductor and the electrical resistance of the second electric conductor is smaller than or equal to 50 µA. By knowing the voltage of the power supply, e.g. the voltage of a battery, the electrical resistance of the first electric conductor and the electrical resistance of the second electric conductor can be determined so to limit the maximum electric current flowing through the first electrode and the second electrode. The sum of the electrical resistance of the first electric conductor and the electrical resistance of the second electric conductor can be equal to the voltage of the power supply divided by the maximum allowed electric current, here: 50 µA.

In an embodiment, the first electrode from its second towards its first free end and the second electrode from its second end towards its first free end are, over its respective length, configured to be during use of the medical device in electric contact with the body tissue. When being inserted, the first electrode and the second electrode forming at least part of the insertable portion, are in electric contact with the body tissue. At least one side of the respective first and second electrode, there is no electric isolation shielding the first and second electrode against the body tissue. On the other hand, the first electric conductor and the second electric conductor can be, over its respective length, configured to be during use of the medical device electrically isolated against the body tissue. Since, during use of the medical device, the first electric conductor and the second electric conductor limit the maximum electric current flowing through the first electrode and the second electrode, the first electric conductor and the second electric conductor should not be in direct electric contact with the body tissue.

According to an embodiment, the first electric conductor comprises a first cable cross-sectional area, the second electric conductor comprises a second cable cross-sectional area, the first electrode comprises a third cable cross-sectional area, and the second electrode comprises a fourth cable cross-sectional area, wherein the first cable cross-sectional area and the second cable cross-sectional area are smaller than the third cable cross-sectional area and the fourth cable cross-sectional area. The electrical resistance of the respective electric conductor or electrode is inter alia dependent on the cross-sectional area, wherein a smaller cross-sectional area provides a higher electrical resistance than a larger cross-sectional area of the same material. For instance, the cross-sectional area of the first electrode and the second electrode can be reduced by laser ablation.

The first cable cross-sectional area can be smaller than the third cable cross-sectional area, and the second cable cross-sectional area can be smaller than the fourth cable cross-sectional area. For instance, the first cable cross-sectional area and the second cable cross-sectional area are of equal size, and/or the third cable cross-sectional area and the fourth cable cross-sectional area are of equal size.

The first cable cross-sectional area and the second cable cross-sectional area can be at least 2-times, at least 4-times, at least 5-times, or at least 10-times smaller than the third cable cross-sectional area and the fourth cable cross-sectional area.

According to an embodiment, the base body comprises a bushing, wherein the electronics unit is arranged on a first side of the bushing and the insertable portion of the insertable analyte sensor is arranged on a second side of the bushing opposite to the first side, wherein the second end of the first electrode electrically connected to the first electric conductor, and the second end of the second electrode electrically connected to the second electric conductor are both located within the bushing or at the first side of the bushing comprising the electronics unit or at the second side of the bushing comprising the insertable portion of the insertable analyte sensor. The bushing has a first side and second side, and provides a channel for the first and second electric conductors and/or the first and second electrode which are electrically connected to the electronics unit. For instance, when being located on the first side or within the bushing, a contact point between the first electrode and the first electric conductor as well as a contact point between the second electrode and the second electric conductor, i.e. the second end of the first electrode and/or the second end of the second electrode, is protected by the base body or the bushing. According to an embodiment, the bushing can be a through hole or duct in the base body allowing the first and second electric conductors and/or the first and second electrode to pass into and out of the base body. Like the base body, the bushing can be made from an electrical isolating material.

Further advantages, features and technical effects of the present invention are apparent from the following description of embodiments and the accompanying figures.
- Figure 1: shows a schematic view of a medical device for detecting at least one analyte in a body fluid according to an embodiment,
- Figure 2: shows a schematic view of a medical device for detecting at least one analyte in a body fluid according to another embodiment, and
- Figure 3: shows a schematic view of a medical device for detecting at least one analyte in a body fluid according to yet another embodiment.

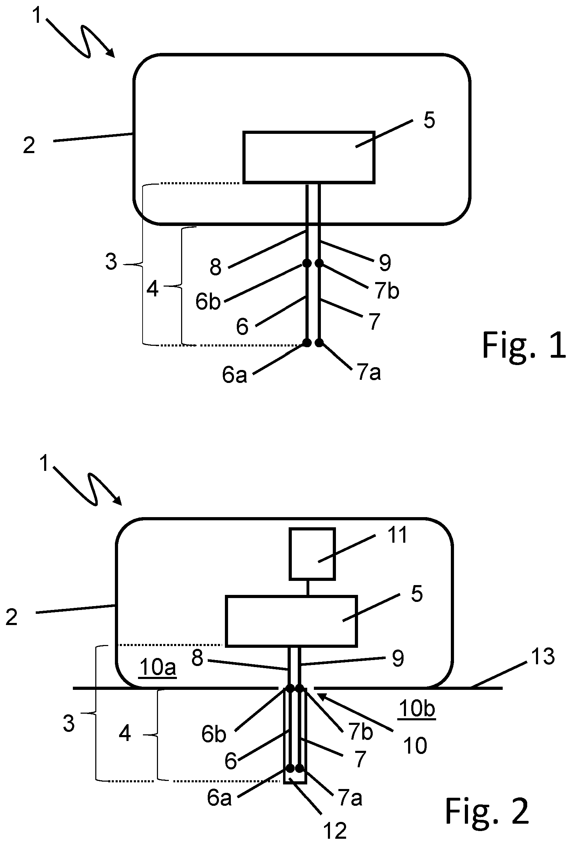

Figure 1 shows a schematic view of a medical device 1. The medical device 1 is configured for detecting at least on analyte in a body fluid. The medical device 1 comprises a base body 2 that is configured for being removably attached to a patient's body. The base body 2 houses an electronics unit 5 that is operably connected to an insertable analyte sensor 3.

The insertable analyte sensor 3 comprises an insertable portion 4 that is configured for being at least partially inserted into body tissue of a user. The insertable portion 4 projects from the base body 2 so that it can be inserted into body tissue by attaching the base body 2 to a skin of a user.

For detecting presence of an analyte in body tissue, the insertable analyte sensor 3 comprises a first electrode 6 and a second electrode 7, both, forming part of the insertable portion 4. The first electrode 6 as well as the second electrode 7 each comprises a first free end 6a, 7a and a second end 6b, 7b. The second end 6b of the first electrode 6 is electrically connected to first electric conductor 8. The electric conductor 8 electrically connects the first electrode 6 to the electronics unit 5. Likewise, the second end 7b of the second electrode 7 is electrically connected to a second electric conductor 9. The second electric conductor 9 electrically connects the second electrode 7 to the electronics unit 5, such that, during use of the medical device 1, an electric current can flow through the first electrode 6 and the second electrode 7.

The first electrode 6 extending from its second end 6b to the first free end 6a of the first electrode 6 as well as the second electrode 7 extending from its second end 7b to the first free end 7a of the second electrode 7 are configured to be, during use of the medical device 1, in electric contact with the body tissue of a user. The first electric conductor 8 and the second electric conductor 9 are not in electric contact with the body tissue, except of being electrically connected to the first electrode 6 and the second electrode 7, respectively.

For mitigating the risk of an uncontrolled flow of electric current in case of a short-circuit failure of the electronic units 5 or part thereof, the electrical resistance of the first electric conductor 8 and the electrical resistance of the second electric conductor 9 is higher than the electrical resistance of the first electrode 6 and the electrical resistance of the second electrode 7.

Figure 2 shows an another embodiment of a medical device 1 for detecting at least one analyte in a body fluid. This embodiment differs from the embodiment of the medical device 1 according to figure 1 in additionally comprising a power supply 11, here a battery. Battery 11 provides electric power to the electronics unit 5. For instance, battery 11 provides 3.4 V. The electronics unit 5 can be a printed circuit board.

The base body 2 further comprises patch 13, wherein patch 13 further comprises an adhesive for removably adhering the medical device 1 by patch 13 to a user's body.

Furthermore, the first electrode 6 and the second electrode 7 are carried by a carrier 12. Carrier 12 supports the first electrode 6 and the second electrode 7, and, thus, provides more stability to the first electrode 6 and the second electrode 7. Carrier 12 projects from the base body 2, and, during use of the medical device 1, is at least partially inserted into body tissue together with the first electrode 6 and the second electrode 7.

Base body 2 further comprises a bushing 10 for allowing the first electric conductor 8 and the second electric conductor 9 to reach from the electronics unit 5 through the base body 2 to the second end 6b of the first electrode 6 and to the second end 7b of the second electrode 7, respectively. The bushing 10 comprises a first side 10a and a second side 10b, wherein on the first side 10a of the bushing 10 there is the electronics unit 5 and wherein on the second side 10b, opposite to the first side 10a, of the bushing 10, there is the insertable portion 4 of the insertable analyte sensor 3.

The transition from the first electric conductor 8 to the first electrode 6 as well as the second electric conductor 9, i.e. the second end 6b of the first electrode 6 and the second end 7b of the second electrode 7, are located within the bushing 10. Thereby, bushing 10 protects the contact point between the first electric conductor 8 and the first electrode 6 as well as the contact point between the second electric conductor 9 and the second electrode 7.

Again, the first electrode 6 extending from its second end 6b to its first free end 6a and the second electrode 7 extending from its second end 7b to its free end 7a are configured to be, during use of the medical device 1, in direct electric contact with the body tissue of a user. The electrical resistance of the first electrode 6 and the electrical resistance of the second electrode 7 each is 9 kOhm, wherein the electrical resistance of the first electric conductor 8 and the electrical resistance of the second electric conductor 9 is each 90 kOhm. Accordingly, the electrical resistance of the first electric conductor 8 and the second electric conductor 9 are equal to each other, and 10-times higher than the electrical resistance of the first electrode 8 and the second electrode 9. The electrical resistance of the first electrode 6 is equal to the electrical resistance of the second electrode 7.

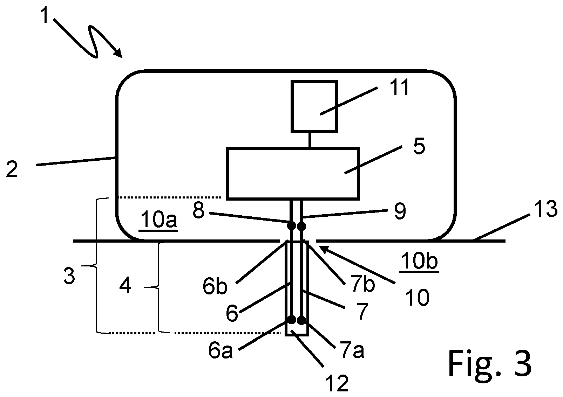

Figure 3 shows a schematic view of another embodiment of a medical device 1 for detecting an analyte in a body fluid. The embodiment as shown in figure 3 differs from the embodiment of a medical device 1 as shown in figure 2 in that the second end 6b of the first electrode 6 and the second end 7b of the second electrode 7 are arranged on the first side 10a of the bushing 10.

### List of reference numbers

- 1: medical device
- 2: base body
- 3: insertable analyte sensor
- 4: insertable portion
- 5: electronics unit
- 6: first electrode
- 6a: first free end of first electrode
- 6b: second end of first electrode
- 7: second electrode
- 7a: first free end of second electrode
- 7b: second end of second electrode
- 8: first electric conductor
- 9: second electric conductor
- 10: bushing
- 10a: first side of bushing
- 10b: second side of bushing
- 11: power supply
- 12: carrier
- 13: patch

## Claims

1. Medical device (1) for detecting at least one analyte in a body fluid, comprising:
• a base body (2) that is configured for reversibly attaching the medical device (1) to a patient's body;
• an insertable analyte sensor (3) comprising an insertable portion (4) that is configured for being at least partially inserted into body tissue of a user; and
• an electronics unit (5) that is operably connected to the insertable analyte sensor (3),
• wherein the insertable analyte sensor (5) comprises a first electrode (6) and a second electrode (7), both, forming part of the insertable portion (4),
• wherein the first electrode (6) comprises a first free end (6a) and a second end (6b), wherein the second end (6b) of the first electrode (6) is electrically connected to a first electric conductor (8) electrically connecting the first electrode (6) of the insertable analyte sensor (3) to the electronics unit (5),
• wherein the second electrode (7) comprises a first free end (7a) and a second end (7b), wherein the second end (7b) of the second electrode (7) is electrically connected to a second electric conductor (9) electrically connecting the second electrode (7) of the insertable analyte sensor (3) to the electronics unit (5), and
• wherein the first electrode (6) extending from the second end (6b) towards its first free end (6a) and the second electrode (7) extending from the second end (7b) towards its first free end (7a) are configured to be, during use of the medical device (1), at least partially in electric contact with the body tissue of a user,
**characterized in that**
• the electrical resistance of the first electric conductor (8) and the electrical resistance of the second electric conductor (9) is higher than the electrical resistance of the first electrode (6) and the electrical resistance of the second electrode (7), wherein the sum of the electrical resistance of first electric conductor (8) and the electrical resistance of the second electric conductor (9) is higher than the sum of the electrical resistance of the first electrode (6) and the electrical resistance of the second electrode (7).

2. Medical device (1) according to claim 1, **characterized in that** the electrical resistance of the first electrode (6) and the electrical resistance of the second electrode (7) are equally high, and/or that the electrical resistance of the first electric conductor (8) and the electrical resistance of the second electric conductor (9) are equally high.

3. Medical device (1) according to any one of claims 1 or 2, **characterized in that** the electrical resistance of the first electric conductor (8) and the electrical resistance of the second electric conductor (9) is at least 2-times, at least 4-times, at least 5-times or at least 10-times higher than the electrical resistance of the first electrode (6) and the electrical resistance of the second electrode (7).

4. Medical device (1) according to any one of claims 1 to 3, **characterized in that** the first electric conductor (8) comprises a first cable cross-sectional area, the second electric conductor (9) comprises a second cable cross-sectional area, the first electrode (6) comprises a third cable cross-sectional area, and the second electrode (7) comprises a fourth cable cross-sectional area, wherein the first cable cross-sectional area and the second cable cross-sectional area are smaller than the third cable cross-sectional area and the fourth cable cross-sectional area.

5. Medical device (1) according to claim 4, **characterized in that** first cable cross-sectional area is smaller than the third cable cross-sectional area, and the second cable cross-sectional area is smaller than the fourth cable cross-sectional area.

6. Medical device (1) according to claim 4 or 5, **characterized in that** the first cable cross-sectional area and the second cable cross-sectional area are of equal size, and/or the third cable cross-sectional area and the fourth cable cross-sectional area are of equal size.

7. Medical device (1) according to any one of claims 4 to 6, **characterized in that** the first cable cross-sectional area and the second cable cross-sectional area are at least 2-times, at least 4-times, at least 5-times, or at least 10-times smaller than the third cable cross-sectional area and the fourth cable cross-sectional area.

8. Medical device (1) according to any one of claims 1 to 7, **characterized in that** the base body (2) comprises a bushing (10), wherein the electronics unit (5) is arranged on a first side (10a) of the bushing (10) and the insertable portion (4) of the insertable analyte sensor (3) is arranged on a second side (10b) of the bushing (10) opposite to the first side (10a), wherein the second end (6b) of the first electrode (6) electrically connected to the first electric conductor (8), and the second end (7b) of the second electrode (7) electrically connected to the second electric conductor (9) are both located within the bushing (10) or at the first side (10a) of the bushing (10) comprising the electronics unit (5) or at the second side (10b) of the bushing (10) comprising the insertable portion (4) of the insertable analyte sensor (3).

9. Medical device (1) according to any one of 1 to 8, **characterized in that** the first electrode (6) from its second end (6b) towards its first free end (6a) and the second electrode (7) from its second end (7b) towards its first free end (7a) are, over its respective length, configured to be during use of the medical device (1) in electric contact with the body tissue.

10. Medical device (1) according to any one of claims 1 to 9, **characterized in that** the first electric conductor (8) and the second electric conductor (9) are, over its respective length, configured to be during use of the medical device (1) electrically isolated against the body tissue.

11. Medical device (1) according to any one of claims 1 to 10, **characterized in that** the medical device (1) comprises a power supply (11) that is configured for providing electric power to the electronics unit (5).

12. Medical device (1) according to claim 11, **characterized in that** the electrical resistance of the first electric conductor (8) and the electrical resistance of the second electric conductor (9) are selected so that the voltage of the power supply (11) divided by the sum of the electrical resistance of the first electrical conductor (8) and the electrical resistance of the second electric conductor (9) is smaller than or equal to 50 µA.

13. Medical device (1) according to any one of claims 1 to 12, **characterized in that** the insertable portion (4) of the insertable analyte sensor (3) comprises a carrier (12) that is configured for carrying the first electrode (6) and the second electrode (7).

14. Medical device (1) according to any one of claims 1 to 13, **characterized in that** the base body (2) comprises a patch (13), wherein the patch (13) comprises an adhesive that is configured for adhering the medical device (1) to a user's body.

## Patentansprüche

1. Medizinische Vorrichtung (1) zum Nachweis von mindestens einem Analyten in einer Körperflüssigkeit, umfassend:
• einen Grundkörper (2), der zur reversiblen Befestigung der medizinischen Vorrichtung (1) am Körper eines Patienten konfiguriert ist;
• einen einführbaren Analytsensor (3), der einen einführbaren Abschnitt (4) umfasst, der dazu konfiguriert ist, zumindest teilweise in Körpergewebe eines Benutzers eingeführt zu werden; und
• eine Elektronikeinheit (5), die mit dem einführbaren Analytsensor (3) funktionsfähig verbunden ist,
• wobei der einführbare Analytsensor (5) eine erste Elektrode (6) und eine zweite Elektrode (7) umfasst, die beide Teil des einführbaren Abschnitts (4) sind,
• wobei die erste Elektrode (6) ein erstes freies Ende (6a) und ein zweites Ende (6b) umfasst, wobei das zweite Ende (6b) der ersten Elektrode (6) elektrisch mit einem ersten elektrischen Leiter (8) verbunden ist, der die erste Elektrode (6) des einführbaren Analytsensors (3) elektrisch mit der Elektronikeinheit (5) verbindet,
• wobei die zweite Elektrode (7) ein erstes freies Ende (7a) und ein zweites Ende (7b) umfasst, wobei das zweite Ende (7b) der zweiten Elektrode (7) elektrisch mit einem zweiten elektrischen Leiter (9) verbunden ist, der die zweite Elektrode (7) des einführbaren Analytsensors (3) elektrisch mit der Elektronikeinheit (5) verbindet, und
• wobei die erste Elektrode (6), die sich vom zweiten Ende (6b) zu ihrem ersten freien Ende (6a) erstreckt, und die zweite Elektrode (7), die sich vom zweiten Ende (7b) zu ihrem ersten freien Ende (7a) erstreckt, dazu konfiguriert sind, während der Verwendung der medizinischen Vorrichtung (1) zumindest teilweise in elektrischem Kontakt mit dem Körpergewebe eines Benutzers zu stehen,
**dadurch gekennzeichnet, dass**
• der elektrische Widerstand des ersten elektrischen Leiters (8) und der elektrische Widerstand des zweiten elektrischen Leiters (9) höher als der elektrische Widerstand der ersten Elektrode (6) und der elektrische Widerstand der zweiten Elektrode (7) sind, wobei die Summe des elektrischen Widerstands des ersten elektrischen Leiters (8) und des elektrischen Widerstands des zweiten elektrischen Leiters (9) höher als die Summe des elektrischen Widerstands der ersten Elektrode (6) und des elektrischen Widerstands der zweiten Elektrode (7) ist.

2. Medizinische Vorrichtung (1) nach Anspruch 1, **dadurch gekennzeichnet, dass** der elektrische Widerstand der ersten Elektrode (6) und der elektrische Widerstand der zweiten Elektrode (7) gleich hoch sind und/oder dass der elektrische Widerstand des ersten elektrischen Leiters (8) und der elektrische Widerstand des zweiten elektrischen Leiters (9) gleich hoch sind.

3. Medizinische Vorrichtung (1) nach einem der Ansprüche 1 oder 2, **dadurch gekennzeichnet, dass** der elektrische Widerstand des ersten elektrischen Leiters (8) und der elektrische Widerstand des zweiten elektrischen Leiters (9) mindestens 2-mal, mindestens 4-mal, mindestens 5-mal oder mindestens 10-mal höher als der elektrische Widerstand der ersten Elektrode (6) und der elektrische Widerstand der zweiten Elektrode (7) sind.

4. Medizinische Vorrichtung (1) nach einem der Ansprüche 1 bis 3, **dadurch gekennzeichnet, dass** der erste elektrische Leiter (8) eine erste Kabelquerschnittsfläche umfasst, der zweite elektrische Leiter (9) eine zweite Kabelquerschnittsfläche umfasst, die erste Elektrode (6) eine dritte Kabelquerschnittsfläche umfasst und die zweite Elektrode (7) eine vierte Kabelquerschnittsfläche umfasst, wobei die erste Kabelquerschnittsfläche und die zweite Kabelquerschnittsfläche kleiner als die dritte Kabelquerschnittsfläche und die vierte Kabelquerschnittsfläche sind.

5. Medizinische Vorrichtung (1) nach Anspruch 4, **dadurch gekennzeichnet, dass** die erste Kabelquerschnittsfläche kleiner als die dritte Kabelquerschnittsfläche ist und die zweite Kabelquerschnittsfläche kleiner als die vierte Kabelquerschnittsfläche ist.

6. Medizinische Vorrichtung (1) nach Anspruch 4 oder 5, **dadurch gekennzeichnet, dass** die erste Kabelquerschnittsfläche und die zweite Kabelquerschnittsfläche gleich groß sind und/oder die dritte Kabelquerschnittsfläche und die vierte Kabelquerschnittsfläche gleich groß sind.

7. Medizinische Vorrichtung (1) nach einem der Ansprüche 4 bis 6, **dadurch gekennzeichnet, dass** die erste Kabelquerschnittsfläche und die zweite Kabelquerschnittsfläche mindestens 2-mal, mindestens 4-mal, mindestens 5-mal oder mindestens 10-mal kleiner als die dritte Kabelquerschnittsfläche und die vierte Kabelquerschnittsfläche sind.

8. Medizinische Vorrichtung (1) nach einem der Ansprüche 1 bis 7, **dadurch gekennzeichnet, dass** der Grundkörper (2) eine Buchse (10) umfasst, wobei die Elektronikeinheit (5) auf einer ersten Seite (10a) der Buchse (10) angeordnet ist und der einführbare Abschnitt (4) des einführbaren Analytsensors (3) auf einer zweiten Seite (10b) der Buchse (10) gegenüber der ersten Seite (10a) angeordnet ist, wobei das zweite Ende (6b) der ersten Elektrode (6), das elektrisch mit dem ersten elektrischen Leiter (8) verbunden ist, und das zweite Ende (7b) der zweiten Elektrode (7), das elektrisch mit dem zweiten elektrischen Leiter (9) verbunden ist, beide innerhalb der Buchse (10) oder auf der ersten Seite (10a) der Buchse (10), die die Elektronikeinheit (5) umfasst, oder auf der zweiten Seite (10b) der Buchse (10), die den einführbaren Abschnitt (4) des einführbaren Analytsensors (3) umfasst, angeordnet sind.

9. Medizinische Vorrichtung (1) nach einem der Ansprüche 1 bis 8, **dadurch gekennzeichnet, dass** die erste Elektrode (6) von ihrem zweiten Ende (6b) zu ihrem ersten freien Ende (6a) und die zweite Elektrode (7) von ihrem zweiten Ende (7b) zu ihrem ersten freien Ende (7a) über ihre jeweilige Länge dazu konfiguriert sind, während der Verwendung der medizinischen Vorrichtung (1) in elektrischem Kontakt mit dem Körpergewebe zu stehen.

10. Medizinische Vorrichtung (1) nach einem der Ansprüche 1 bis 9, **dadurch gekennzeichnet, dass** der erste elektrische Leiter (8) und der zweite elektrische Leiter (9) über ihre jeweilige Länge dazu konfiguriert sind, während der Verwendung der medizinischen Vorrichtung (1) elektrisch gegen das Körpergewebe isoliert zu sein.

11. Medizinische Vorrichtung (1) nach einem der Ansprüche 1 bis 10, **dadurch gekennzeichnet, dass** die medizinische Vorrichtung (1) eine Stromversorgung (11) umfasst, die zum Bereitstellen von elektrischem Strom für die Elektronikeinheit (5) konfiguriert ist.

12. Medizinische Vorrichtung (1) nach Anspruch 11, **dadurch gekennzeichnet, dass** der elektrische Widerstand des ersten elektrischen Leiters (8) und der elektrische Widerstand des zweiten elektrischen Leiters (9) so ausgewählt sind, dass die Spannung der Stromversorgung (11) geteilt durch die Summe des elektrischen Widerstands des ersten elektrischen Leiters (8) und des elektrischen Widerstands des zweiten elektrischen Leiters (9) kleiner als oder gleich 50 µA ist.

13. Medizinische Vorrichtung (1) nach einem der Ansprüche 1 bis 12, **dadurch gekennzeichnet, dass** der einführbare Abschnitt (4) des einführbaren Analytsensors (3) einen Träger (12) umfasst, der zum Tragen der ersten Elektrode (6) und der zweiten Elektrode (7) konfiguriert ist.

14. Medizinische Vorrichtung (1) nach einem der Ansprüche 1 bis 13, **dadurch gekennzeichnet, dass** der Grundkörper (2) ein Pflaster (13) umfasst, wobei das Pflaster (13) einen Klebstoff umfasst, der zum Anhaften der medizinischen Vorrichtung (1) am Körper eines Benutzers konfiguriert ist.

## Revendications

1. Dispositif médical (1) pour détecter au moins un analyte dans un fluide corporel, comprenant :
• un corps de base (2) qui est conçu pour attacher de manière réversible le dispositif médical (1) au corps d'un patient ;
• un capteur d'analyte insérable (3) comprenant une partie insérable (4) qui est conçue pour être au moins partiellement insérée dans un tissu corporel d'un utilisateur ; et
• une unité électronique (5) qui est connectée de manière fonctionnelle au capteur d'analyte insérable (3),
• dans lequel le capteur d'analyte insérable (5) comprend une première électrode (6) et une seconde électrode (7), toutes deux formant une partie de la partie insérable (4),
• dans lequel la première électrode (6) comprend une première extrémité libre (6a) et une seconde extrémité (6b), dans lequel la seconde extrémité (6b) de la première électrode (6) est connectée électriquement à un premier conducteur électrique (8) connectant électriquement la première électrode (6) du capteur d'analyte insérable (3) à l'unité électronique (5),
• dans lequel la seconde électrode (7) comprend une première extrémité libre (7a) et une seconde extrémité (7b), dans lequel la seconde extrémité (7b) de la seconde électrode (7) est connectée électriquement à un second conducteur électrique (9) connectant électriquement la seconde électrode (7) du capteur d'analyte insérable (3) à l'unité électronique (5), et
• dans lequel la première électrode (6) s'étendant depuis la seconde extrémité (6b) vers sa première extrémité libre (6a) et la seconde électrode (7) s'étendant depuis la seconde extrémité (7b) vers sa première extrémité libre (7a) sont conçues pour être, pendant l'utilisation du dispositif médical (1), au moins partiellement en contact électrique avec le tissu corporel d'un utilisateur,
**caractérisé en ce que**
• la résistance électrique du premier conducteur électrique (8) et la résistance électrique du second conducteur électrique (9) sont supérieures à la résistance électrique de la première électrode (6) et la résistance électrique de la seconde électrode (7), dans lequel la somme de la résistance électrique du premier conducteur électrique (8) et de la résistance électrique du second conducteur électrique (9) est supérieure à la somme de la résistance électrique de la première électrode (6) et de la résistance électrique de la seconde électrode (7).

2. Dispositif médical (1) selon la revendication 1, **caractérisé en ce que** la résistance électrique de la première électrode (6) et la résistance électrique de la seconde électrode (7) sont élevées de manière égale, et/ou **en ce que** la résistance électrique du premier conducteur électrique (8) et la résistance électrique du second conducteur électrique (9) sont élevées de manière égale.

3. Dispositif médical (1) selon l'une quelconque des revendications 1 ou 2, **caractérisé en ce que** la résistance électrique du premier conducteur électrique (8) et la résistance électrique du second conducteur électrique (9) sont au moins 2 fois, au moins 4 fois, au moins 5 fois ou au moins 10 fois supérieures à la résistance électrique de la première électrode (6) et la résistance électrique de la seconde électrode (7).

4. Dispositif médical (1) selon l'une quelconque des revendications 1 à 3, **caractérisé en ce que** le premier conducteur électrique (8) comprend une première zone de section transversale de câble, le second conducteur électrique (9) comprend une deuxième zone de section transversale de câble, la première électrode (6) comprend une troisième zone de section transversale de câble, et la seconde électrode (7) comprend une quatrième zone de section transversale de câble, dans lequel la première zone de section transversale de câble et la deuxième zone de section transversale de câble sont inférieures à la troisième zone de section transversale de câble et la quatrième zone de section transversale de câble.

5. Dispositif médical (1) selon la revendication 4, **caractérisé en ce que** la première zone de section transversale de câble est inférieure à la troisième zone de section transversale de câble, et la deuxième zone de section transversale de câble est inférieure à la quatrième zone de section transversale de câble.

6. Dispositif médical (1) selon la revendication 4 ou 5, **caractérisé en ce que** la première zone de section transversale de câble et la deuxième zone de section transversale de câble sont de taille égale, et/ou la troisième zone de section transversale de câble et la quatrième zone de section transversale de câble sont de taille égale.

7. Dispositif médical (1) selon l'une quelconque des revendications 4 à 6, **caractérisé en ce que** la première zone de section transversale de câble et la deuxième zone de section transversale de câble sont au moins 2 fois, au moins 4 fois, au moins 5 fois ou au moins 10 fois inférieures à la troisième zone de section transversale de câble et la quatrième zone de section transversale de câble.

8. Dispositif médical (1) selon l'une quelconque des revendications 1 à 7, **caractérisé en ce que** le corps de base (2) comprend une bague de raccordement (10), dans lequel l'unité électronique (5) est agencée sur un premier côté (10a) de la bague de raccordement (10) et la partie insérable (4) du capteur d'analyte insérable (3) est agencée sur un second côté (10b) de la bague de raccordement (10) opposé au premier côté (10a), dans lequel la seconde extrémité (6b) de la première électrode (6) connectée électriquement au premier conducteur électrique (8), et la seconde extrémité (7b) de la seconde électrode (7) connectée électriquement au second conducteur électrique (9) sont toutes deux situées à l'intérieur de la bague de raccordement (10) ou au niveau du premier côté (10a) de la bague de raccordement (10) comprenant l'unité électronique (5) ou au niveau du second côté (10b) de la bague de raccordement (10) comprenant la partie insérable (4) du capteur d'analyte insérable (3).

9. Dispositif médical (1) selon l'une quelconque des revendications 1 à 8, **caractérisé en ce que** la première électrode (6) depuis sa seconde extrémité (6b) vers sa première extrémité libre (6a) et la seconde électrode (7) depuis sa seconde extrémité (7b) vers sa première extrémité libre (7a) sont, sur leur longueur respective, conçues pour être pendant l'utilisation du dispositif médical (1) en contact électrique avec le tissu corporel.

10. Dispositif médical (1) selon l'une quelconque des revendications 1 à 9, **caractérisé en ce que** le premier conducteur électrique (8) et le second conducteur électrique (9) sont, sur leur longueur respective, conçus pour être pendant l'utilisation du dispositif médical (1) isolés électriquement du tissu corporel.

11. Dispositif médical (1) selon l'une quelconque des revendications 1 à 10, **caractérisé en ce que** le dispositif médical (1) comprend une alimentation électrique (11) qui est conçue pour fournir de l'énergie électrique à l'unité électronique (5).

12. Dispositif médical (1) selon la revendication 11, **caractérisé en ce que** la résistance électrique du premier conducteur électrique (8) et la résistance électrique du second conducteur électrique (9) sont choisies de telle sorte que la tension de l'alimentation électrique (11) divisée par la somme de la résistance électrique du premier conducteur électrique (8) et de la résistance électrique du second conducteur électrique (9) est inférieure ou égale à 50 µA.

13. Dispositif médical (1) selon l'une quelconque des revendications 1 à 12, **caractérisé en ce que** la partie insérable (4) du capteur d'analyte insérable (3) comprend un support (12) qui est conçu pour porter la première électrode (6) et la seconde électrode (7).

14. Dispositif médical (1) selon l'une quelconque des revendications 1 à 13, **caractérisé en ce que** le corps de base (2) comprend un timbre (13), dans lequel le timbre (13) comprend un adhésif qui est conçu pour faire adhérer le dispositif médical (1) au corps d'un utilisateur.
